# EUROPEAN PATENT APPLICATION

(11) **EP 4 379 055 A1**
(43) Date of publication of application: **05.06.2024**
(21) Application number: 22849769.9
(22) Date of filing: 14.07.2022
(51) Int. Cl.: C12N 15/63, C12N 15/67, C07K 14/005, C07K 14/00

(54) **MRNA FOR PROTEIN EXPRESSION AND TEMPLATE THEREFOR**

(30) Priority: 27.07.2021 KR 20210098684; 20.05.2022 KR 20220062306
(71) Applicant: SK Bioscience Co., Ltd., Bundang-gu, Seongnam-si, Gyeonggi-do, 13494 (KR)
(72) Inventor: SHIN, Jin Hwan, Seongnam-si, Gyeonggi-do 13494 (KR); CHOI, Young Joon, Seongnam-si, Gyeonggi-do 13494 (KR); KIM, Hun, Seongnam-si, Gyeonggi-do 13494 (KR); KIM, Min Ji, Seongnam-si, Gyeonggi-do 13494 (KR); LEE, Jeong Min, Seongnam-si, Gyeonggi-do 13494 (KR)
(74) Representative: Weickmann & Weickmann PartmbB
(86) International application number: PCT/KR2022/010285
(87) International publication number: WO 2023/008793

(57) **Abstract**

The present application relates to mRNA for protein expression and a template therefor, and provides an mRNA transcription vector including a gene construct according to an embodiment, a method of producing an mRNA molecule including the processes of performing *in vitro* transcription using the mRNA transcription vector, an mRNA molecule prepared by the method, and an immunogenic composition including the mRNA molecule as an active ingredient.

## Description

### Technical Field

The present disclosure relates to mRNA for protein expression and a template therefor. This patent application claims priority to Korean Patent Application No. 10-2021-0098684, filed on July 27, 2021, with the Korean Intellectual Property Office, and Korean Patent Application No. 10-2022-0062306, filed on May 20, 2022, with the Korean Intellectual Property Office, the disclosures of which are incorporated by reference herein.

### Background Art

Nucleic acid-based treatment and vaccine development technology has shown insufficient effectiveness as a therapeutic agent due to insufficient transcription and translation efficiency when applied to a target organism beyond the cellular level, which has been pointed out as a limitation in the development of nucleic acid-based therapeutic agents. Therefore, increasing the ability to express an antigenic protein in/out of a cell for treatment of an infectious disease is one of the essential requirements for the development of a pharmaceutical using an artificial nucleic acid molecule. In addition, in the field of nucleic acid-based treatment and vaccine development, securing an mRNA sequence with high stability and translation efficiency has emerged as an essential condition for mRNA-based therapeutic agents.

On the other hand, an mRNA vaccine refers to a pharmaceutical used for the prevention and treatment of cancer, infectious diseases, autoimmune diseases, etc., using a protein encoded by mRNA as an antigen. Compared to a DNA vaccine, the mRNA vaccine has an advantage of being stable and easy to mass produce, and is expected to be widely used as a platform for anti-cancer vaccines and infectious disease vaccines in a pandemic situation in the future. The mRNA vaccine includes an artificial RNA molecule produced by mimicking a natural mRNA structure as an active ingredient, and its main goal is to strengthen the immune system of an individual by using the RNA molecule. Currently, mRNA-1273 of Moderna and BNT162b2 of Biontech/Pfizer, etc. have been commercialized through the emergency use approval process, but these mRNA vaccines essentially need technical elements for delivery to the cytoplasm due to the instability of RNA molecules, and after delivering RNA molecules to the cytoplasm, damage to RNA molecules or decreases in translation efficiency due to excessive innate immune responses need to be minimized, thus multifaceted research is required in the field of mRNA vaccine technology. In particular, it is known that the immune activation properties of naked mRNA, which inherently induces the stimulation of an adjuvant such as a toll-like receptor agonist (TLR agonist), conversely interferes with the transcription signaling system of the mRNA, thereby inhibiting the expression of sufficient amounts of the mRNA antigen to exhibit pharmacological activity. To overcome these problems, attempts are actively underway to produce a vaccine using modified mRNA from which immunogenicity has been removed or attenuated. However, in this case, due to the low immunogenicity of mRNA, there are limitations in inducing effective humoral immunity and/or cellular immune responses.

Under this technical background, as part of the development to improve the efficacy of mRNA molecules used medicinally, multifaceted research is being conducted to improve the expression of mRNA molecules or to increase their immunogenicity (Korean public patent 10-2022-0017377), which is still incomplete.

### Disclosure

### Technical Problem

One aspect is to provide an mRNA transcription vector including a promoter region recognized by an RNA polymerase and a gene construct that may improve expression of a target mRNA antigen.

Another aspect is to provide a method of producing an mRNA molecule utilizing an mRNA transcription vector as a template, and an mRNA molecule prepared by the method.

Another aspect is to provide an immunogenic composition including the mRNA molecule and a pharmaceutically acceptable excipient as an active ingredient.

Other objectives and advantages of the present application will become apparent from the following detailed description, together with the appended claims and drawings. Anything not set forth herein will be readily recognized and inferred by one skilled in the art or similar art and is hereby omitted.

### Technical Solution

One aspect is an mRNA transcription vector including a promoter region recognized by an RNA polymerase and a gene construct operably linked to the promoter region,
wherein the gene construct provides an mRNA transcription vector including a 5'-untranslated region (5'-UTR) area consisting of SEQ ID NO: 1 or a nucleotide sequence that has 90% or more sequence identity thereto; an open reading frame (ORF) region including a nucleotide sequence encoding a target antigen, operably linked to the 5'-UTR region; and a 3'-untranslated region (3'-UTR) area including two repeats of a monomeric sequence consisting of SEQ ID NO: 2 or a nucleotide sequence that has 90% or more sequence identity thereto, operably linked to the open reading frame region.

As used herein, the term "nucleotide sequence" refers to a polymeric substance including a plurality of nucleotide monomers, more specifically, a polymer in which the plurality of nucleotide monomers are linked together by phosphodiester bonds of a sugar/phosphate backbone, and may be used interchangeably with the terms "polynucleotide", "nucleic acid", and "nucleic acid molecule". The polynucleotide is a biopolymer essential to life, which may be RNA or DNA that encodes genetic information through a unique nucleic acid sequence. The polynucleotide may be isolated, artificially synthesized, or non-naturally occurring or engineered, wherein "non-naturally occurring or engineered" refers to a state produced by artificial modification rather than a state of existence that occurs in a natural state. Here, the artificial modification may be intended to improve the expression of the target antigen in a cell by mimicking the structure of natural mRNA, specifically, mature mRNA.

As used herein, the term "mRNA (Messenger RNA)" refers to an RNA that is transcribed from a DNA template and deliver the genetic information of the DNA to ribosomes in the cytoplasm. The process of transcription in a eukaryotic organism take place within the nucleus of the cell and include the procedure of processing a premature RNA. Specifically, this process is referred to as post-transcriptional modification and include the process of splicing, 5'-capping, polyadenylation, and export from the nucleus or mitochondria, etc. As a result of this process, mature mRNA which include a nucleotide sequence that may be translated into the amino acid sequence of a specific peptide or protein is produced. Typically, the mature mRNA may optionally include a 5'-cap, a 5'UTR, an open reading frame, a 3'UTR, and a poly A tail. The mRNA may be synthesized according to any of several disclosed methods, for example, the mRNA may be synthesized by *in vitro* transcription (IVT).

As used herein, the term "vector" refers to a vector that may express a target antigen in a suitable host cell, and a genetic construct including regulatory elements operably linked to cause the gene insert to be expressed. The Vector according to an embodiment may include an expression regulatory element such as a promoter, operator, initiation codon, termination codon, polyadenylation signal, and/or enhancer, and the promoter of the vector may be constitutive or inducible. In addition, the vector may be an expression vector, which may stably express the target antigen in a host cell. The expression vector may be typical ones used in the art for expressing a foreign protein in a plant, animal or microorganism, and the vector may be produced by various methods known in the art. In the vector, the aforementioned gene construct sequence may be operably linked to a promoter. The term, "operably linked" may refer to the linkage of nucleotide sequences on a single nucleic acid fragment such that the function of one is affected by the other. In an embodiment, the vector may be a vector that may express an mRNA molecule as a target antigen in a host cell, in other words, may refer to an mRNA transcription vector. The mRNA transcription vector may consist of a genetic construct in the form of DNA, and may refer to template DNA for mRNA production. To this end, the mRNA transcription vector may include a promoter recognized by an RNA polymerase, which mediates a transcription process to produce an mRNA molecule.

In an embodiment, the mRNA transcription vector may be in plasmid form, and in addition, the mRNA transcription vector may be a linearized vector, specifically, a linearized plasmid. The linearized plasmid may be obtained by contacting the plasmid DNA with a restriction enzyme under suitable conditions wherein the restriction enzyme cuts the plasmid DNA at the recognition site and disrupts the plasmid structure. The linearized plasmid may include a free 5' end, and a free 3' end, for carrying out the subsequent process of *in vitro* transcription. Additionally, in the plasmid, known techniques in the art may be applied without limitation to factors such as the type of plasmid and restriction enzyme recognition site, etc.

As used herein, the term "RNA polymerase" refers to an enzyme that synthesizes primary transcript RNA from DNA. The RNA polymerase may be, for example, a T7 RNA polymerase, a T3 RNA polymerase, an SP6 RNA polymerase, or a mitochondrial polymerase (POLRMT). The term "promoter region recognized by an RNA polymerase" may refer to a region of DNA sequence that serves as a template that may produce a promoter mRNA that is recognized by an RNA polymerase by the transcriptional process described above.

In an embodiment, the promoter region may be recognized by an RNA polymerase acting in the cytoplasm, for example, recognized by a T7 RNA polymerase, and may consist of a nucleotide sequence of SEQ ID NO: 4 or SEQ ID NO: 15, or a sequence that has at least 90% or more sequence identity to the nucleotide sequence.

As used herein, the term "identity" refers to the overall relatedness between polymer molecules, for example, between nucleic acids (for example, DNA molecules and/or RNA molecules) and/or between polypeptides. For example, polypeptides are considered "substantially identical" to each other if their amino acid sequences are at least, 60%, 65%, 70%, 75%, 80%, 85%, 90%, 95%, or 99% identical. Calculation of the percentage identity of two nucleic acid or polypeptide sequences may be performed, for example, by aligning the two sequences for optimal comparison purposes (for example, gaps may be introduced in one or both of the first and second sequences for optimal alignment, and non-identical sequences may be ignored for comparison purposes). For example, the length of the aligned sequence for comparison purposes is at least 60%, at least 70%, at least 80%, at least 90%, at least 95%, or substantially 100% of the length of the reference sequence. Next, the nucleic acid or polypeptide sequences at the corresponding positions are compared. Determination of percent identity between two sequences and comparison of sequences may be accomplished using mathematical algorithms. As is well known to those skilled in the art, amino acid or nucleic acid sequences may be compared using any of a variety of algorithms, including those available in commercial computer programs such as BLASTN for nucleotide sequences and BLASTP, gapped BLAST, and PSIBLAST for amino acid sequences.

In the present disclosure, unless otherwise stated regarding the position of the nucleotide sequence, the present disclosure indicates that the nucleotide sequence is linked or located in the direction from the 5' end to the 3' end.

In addition, as a technical element to improve the expression of an mRNA molecule or an mRNA antigen, the gene construct may include a 5'-untranslated region area; an open reading frame region including a nucleotide sequence encoding a target antigen; and a 3'-untranslated region area.

As used herein, the term "open reading frame" may generally be a sequence of triplets of nucleotides that may be translated into a peptide or protein, and may be used interchangeably with the term "protein encoding region". The open reading frame may preferably include a start codon, in other words, a combination of three nucleotide sequences (ATG or AUG) encoding the amino acid methionine, in a subsequent region beginning at the 5'-end thereof, and a stop codon (for example, TAA, TAG, TGA, or UAA, UAG, UGA) directing termination of translation, preferably in a region toward the 3'-end thereof. The term, "open reading frame region" may refer to a region of DNA sequence that serves as a template that may produce an open reading frame mRNA, in other words, a target mRNA antigen, by the transcriptional process described above.

As used herein, the term "5'-Untranslated region" refers to a region located at the 5' end of the open reading frame, specifically, a region of mRNA upstream from the initiation codon. As used herein, the term "5'-Untranslated region area" may refer to a region of DNA sequence that serves as a template from which a 5'-Untranslated region mRNA may be produced by the transcriptional process described above.

As used herein, the term "3'-Untranslated region (3'-UTR)" refers to a region located at the 3' end of the open reading frame, specifically, a region of mRNA downstream from a stop codon. The term "3'-untranslated region area" may refer to a region of DNA sequence that serves as a template from which a 3'-untranslated region mRNA may be produced by the transcriptional process described above.

In an embodiment, the open reading frame region may include a nucleotide sequence encoding an antigen derived from a pathogen. The pathogen may be selected from the group consisting of a virus, a bacterium, a prion, a fungus, a protozoon, a viroid, and a parasite, but is not limited thereto. For example, the open reading frame region may be a nucleotide sequence encoding a virus surface protein or a functional domain thereof, but may be applied without limitation as long as it is an mRNA antigen that may cause pathological symptoms by infecting mammals, including humans.

In an embodiment, the 5'-untranslated region area is a modification of a sequence derived from human hemoglobin subunit alpha 2 (HBA2), which may consist of the nucleotide sequence of SEQ ID NO: 1 or a sequence that has at least 90% or more sequence identity to the nucleotide sequence.

In an embodiment, the 3'-untranslated region area may be a modification of a sequence derived from human hemoglobin subunit beta (HBA2), including two repeats of the nucleotide sequence of SEQ ID NO: 2 or a monomeric sequence consisting of a sequence that has at least 90% or more sequence identity to the nucleotide sequence. Wherein the 3'-untranslated region area may consist of a nucleotide sequence to which the monomeric sequence is directly linked, or linked by a linker sequence. In addition, the 3'-untranslated region area may consist of a nucleotide sequence of SEQ ID NO: 3 or a sequence that has at least 90% or more sequence identity to the nucleotide sequence.

In addition, the gene construct may additionally include a nucleotide sequence operably linked to the 5'-UTR, transcribed as a 5' Cap, and/or a nucleotide sequence operably linked to the 3'-UTR region, transcribed as a poly A tail.

As used herein, the term "5'Cap" refers to a structure located at the 5'-end region that affects the stability and expression efficiency of a polynucleotide, which may be formed by a modified nucleotide, typically a derivative of a guanine nucleotide. The 5'cap may be any one of m⁷G(5')ppp(5')(2'OMeA)pG, m⁷Gppp, Gppp, m'(3'OMeG)(5')ppp(5')(2'OMeA)pG, m⁷(3'OMeG)(5')ppp(5')(2'OMeG)pG, G(5')ppp(5')G, m⁷G(5')ppp(5')G, 3'-O-Me-m⁷G(5')ppp(5')G, m⁷G(5')ppp(5')(2'OMeA)pG, or m7G(5')ppp(5')(2'OMeA)pU, but a known 5'cap having the same functionality as above may be applied without limitation.

As used herein, the term "poly A tail" refers to a structure located in the 3'-end region that delays the degradation process of an RNA exo-nuclease and affects the stability and *in vivo* half-life of a polynucleotide, thereby affecting its expression efficiency, and may generally be formed by a plurality of adenine nucleotide sequences. In an embodiment, the poly A tail may consist of 20 to 200 adenine sequences, for example, may be 20 to 190, 20 to 170, 20 to 150, 20 to 130, 20 to 110, 20 to 90, 20 to 70, 20 to 50, 20 to 30, 30 to 190, 30 to 170, 30 to 150, 30 to 130, 30 to 110, 30 to 90, 30 to 70, or 30 to 50 repetitive adenine nucleotide sequences. In addition, the poly A tail may consist of a plurality of monomers connected by linkers.

In an embodiment, the mRNA transcription vector includes a promoter region consisting of a nucleotide sequence of SEQ ID NO: 4 or SEQ ID NO: 15 and a gene construct, wherein the gene construct may be a plasmid including a 5'-UTR region consisting of a nucleotide sequence of SEQ ID NO: 1; an ORF region operably linked to the 5'-UTR region; and a 3'-UTR region consisting of a nucleotide sequence of SEQ ID NO: 3.

According to an embodiment, an mRNA transcription vector according to an embodiment including a gene construct that has a certain combination of the 5'-UTR region and the 3'-UTR region was able to express luciferase mRNA at a higher efficiency compared to a typical vector. In addition, the mRNA molecule according to an embodiment prepared by adopting the coronavirus spike protein as the target mRNA antigen induced a high level of immune response when administered to an animal model in the form of a vaccine formulation, and also showed effective immunogenicity against mRNA antigens including various conformational variants, and the mRNA transcription vectors may be utilized in the field of production of the mRNA molecule and therapeutic agent or vaccine, etc. including the mRNA molecule.

Another aspect provides a method for producing an mRNA molecule including the processes of performing *in vitro* transcription using the mRNA transcription vector as a template, and an mRNA molecule prepared by the method.

The following terms or elements referred to in the method of producing the mRNA molecule and the mRNA molecule prepared by the method are the same as those referred to in the above description of the mRNA transcription vector.

As used herein, the term "*in vitro* transcription" refers to a process in which a target mRNA molecule is synthesized in a cell-free system (*in vitro*), preferably in which the DNA consisting the transcription vector may be used as a template for the production of the mRNA transcript, and RNA polymerase may be used to control the *in vitro* transcription process. In general, a DNA template for RNA of *in vitro* RNA transcription may be obtained by cloning a specific cDNA corresponding to each RNA to be transcribed *in vitro* and introducing it into a suitable vector for RNA of *vitro* transcription.

In an embodiment, the process of performing the in vitro transcription may be to use an mRNA transcription vector according to an embodiment including a gene construct that has a combination of the specific 5'-UTR region and 3'-UTR region described above, and other conditions of performance, or the composition of the ORF region, may be varied according to the purpose.

Another aspect provides an immunogenic composition including the mRNA molecule and a pharmaceutically acceptable excipient as an active ingredient, an mRNA transcription vector for producing the immunogenic composition or a medicamental use of the mRNA molecule produced by the transcription vector, or a method of stimulating an immune response including the process of administering the immunogenic composition to an individual.

Among the terms or elements mentioned in the immunogenic composition below, those mentioned in the description of the mRNA transcription vector, the method of producing the mRNA molecule, and the mRNA molecule prepared by the method are as described above.

As used herein, the term "immunogenic composition" refers to a substance containing an active ingredient, or an effective amount thereof, effective to induce a specific degree of immunity in a subject against a specific pathogen or disease, and may be used interchangeably with the terms "vaccine", "vaccine formulation", and "vaccine composition". In addition, an immunogenic composition may be a pharmaceutical composition that induces a reduction in the severity, duration, or other manifestation of symptoms associated with a disease or infection by a pathogen. Thus, the immunogenic composition may optionally include a pharmaceutically acceptable carrier, diluent, excipient, buffer, salt, surfactant, cryoprotectant, etc.

As used herein, the term "immunogenicity" refers to the ability of a composition to elicit an immune response against a specific pathogen, which immune response may be a cellular immune response mediated primarily by cytotoxic T-cells and cytokine-producing T-cells, or a humoral immune response mediated primarily by helper T-cells that then activate B-cells to produce antibodies.

As used herein, the term "pharmaceutically acceptable excipient" may include any substance that, when combined/mixed with the mRNA molecule, maintains the activity of the mRNA molecule and does not react with the subject's immune system. Examples include, but are not limited to, buffer systems such as phosphate buffered saline, surfactant, emulsions such as water, oil/water emulsion, and various forms of lubricant, and any standard pharmaceutical excipient such as starch, milk, sugar, certain forms of clay, gelatin, stearic acid or its salt, magnesium or calcium stearate, talc, vegetable oil, gum, glycol, or other known excipient.

The immunogenic composition may be in any form known in the art, for example, but is not limited to a form of solution and injectable formulation. In the case of solution or injectable formulation, the immunogenic composition may contain 10 % to 40 % propylene glycol, etc., if necessary. The solution or injectable formulation form may include any diluent or buffer known in the art. In addition, the immunogenic composition may be prepared just prior to use by preserving the formulation including the active ingredient in a container, such as a vial, and adding the necessary carrier or azubant, saline, etc. for an injectable formulation prior to use.

In an embodiment, the mRNA molecules may form a complex with at least one or more lipid component to form liposomes, lipid nanoparticles, and/or lipoplexes.

In an embodiment, the lipid nanoparticles may include an ionizable cationic lipid as one component, and may also include other ingredients such as a helper lipid, stabilizer to encapsulate mRNA or to aid in delivery efficiency, stabilization, etc.

In an embodiment, the lipid nanoparticle may include a cationic lipid and a neutral lipid (for example, a phospholipid), sterol or steroid (for example, cholesterol), PEG-conjugated lipid, etc. At this time, a specific embodiment of the cationic lipid may use ALC-0315([(4-hydroxybutyl)azanediyl]di(hexane-6,1-diyl) bis(2-hexyldecanoate)), SM-102(9-Heptadecanyl 8-{(2-hydroxyethyl)[6-oxo-6-(undecyloxy)hexyl]amino}octanoate), N,N-dioleyl-N,N-dimethylammonium chloride (DODAC), N-(I-(2,3-dioleyloxy)propyl)-N,N,N-trimethylammonium chloride(DOTMA), N-(I-(2,3-dioleoyloxy)propyl)-N,N,N-trimethylammonium chloride (DOTAP), N,N-dimethyl-(2,3-dioleyloxy)propylamine (DODMA), N,N-distearyl-N,N-dimethylammonium bromide(DDAB), 1,2-dilinoleyloxy-N,N-dimethylaminopropane(DLinDMA), 1,2-dilinolenyloxy-N,Ndimethylaminopropane (DLenDMA), DLin-KC2-DMA, (6Z,9Z,28Z,31Z)-Heptatriaconta-6,9,28,31-tetraen-19-yl 4-(dimethylamino)butanoate (DLinMC3-DMA, CAS 1224606-06-7), ((4-hydroxybutyl)azanediyl)bis(hexane-6,1-diyl)bis(2-hexyldecanoate), etc. but are not particularly limited thereto.

In an embodiment, the lipid nanoparticle may include, a cationic lipid such as those described above (ranging from 35 mol% to 60 mol% of overall lipids), phospholipid (ranging from 5 mol% to 15 mol% of overall lipids), cholesterol (ranging from 30 mol% to 50 mol% of overall lipids), and PEG-conjugated lipids such as DMG-PEG2000 (ranging from 0.5 mol% to 2.5 mol% of overall lipids), but are not particularly limited thereto.

In an embodiment, the lipid nanoparticle may apply an ingredient known in the art and may include a cationic lipid, helper lipid, and PEG-conjugated lipid. Here, the cationic lipid may be, for example, ALC-0315 and/or SM-102; the helper lipid may be distearoylphosphatidylcholine (DSPC) and/or cholesterol; and the PEG-conjugated lipid may be ALC-0159, and/or PEG-dimyristoyl glycerol (PEG-DMG), for example, may be a particle consisted of ALC-0315: DSPC: Chol: ALC-0159 (47.5:10:40.8:1.7 mol%), but is not limited thereto.

In an embodiment, the lipid compounds disclosed in WO2014/172045A1, WO2020/252589A1, and WO2021/000041A1 may be used as the lipid of the lipid nanoparticle without any particular limitation, and PNI123/DSPC/cholesterol/PEG-DMG (50:10:38.5 or 37.5:1.5 or 2.5 mol%), PNI123/DOPE/cholesterol/PEG-DMG may be used, but are not limited thereto.

As used herein, the term "individual" refers to a person in need of treatment or prevention of a disease, and more specifically, a mammal, such as a human or non-human primate, mouse, dog, cat, horse, and bovine, etc.

### Advantageous Effects

According to the mRNA transcription vector according to an aspect, and the method for producing an mRNA molecule using the transcription vector, a desired mRNA molecule may be expressed with high efficiency by including a specific gene construct. In addition, it has been confirmed that the mRNA molecule prepared by the above method induce a high level of immune response in an animal model, and may be applied to the treatment or prevention of various infectious diseases.

Therefore, the mRNA molecule-related technology according to an aspect may be utilized in the field of producing mRNA molecules and therapeutic agents or vaccines, etc. including the mRNA molecule.

### Description of Drawings

FIG. 1 is a diagram briefly illustrating the structure of an mRNA transcription vector according to an embodiment.
FIG. 2 is a diagram briefly illustrating the structure of an mRNA transcription vector produced to compare intracellular antigen expression levels with an mRNA transcription vector according to an embodiment, wherein A of FIG. 2 illustrates pSKBS-Luciferase, B of FIG. 2 illustrates pMod-Luciferase, and C of FIG. 2 illustrates pBNT-Luciferase.
FIG. 3 shows the result of confirming the activity of luciferase after transfection of a luciferase mRNA molecule prepared using an mRNA transcription vector according to an embodiment into an HEK 293 cell.
FIG. 4 shows the results of, after administering an immunogenic composition including a spike mRNA molecule in a coronavirus to a mouse, confirming the antibody titer of IgG in the serum of the mouse induced by the immunogenic composition, according to an embodiment.
FIG. 5 shows the results of, after administering an immunogenic composition including a full-length Spike mRNA molecule (Spike) or RBD mRNA molecule (RBD) in a coronavirus to a mouse, comparing antibody titers of IgG in the serum of the mouse induced by the composition, according to an embodiment.
FIG. 6 shows the results of, after administering to a mouse an immunogenic composition including a spike mRNA molecule with a modified poly-A tail sequence, comparing the antibody titers of IgG in the serum of the mouse induced by the composition, according to an embodiment.
FIG. 7 shows the results of, after administering to a mouse an immunogenic composition including an mRNA molecule with a modified uridine sequence in a spiked mRNA molecule, comparing the antibody titers of IgG in the serum of the mouse induced by the composition, according to an embodiment.
FIG. 8 shows the results of confirming changes in the ratio of CD103+, CD62L^{high}, CD44^{high}, and CD4+ T cells in response to external antigen stimulation in animal models (G1 to G5) to which each vaccine formulation was administered, according to an embodiment.
FIG. 9 shows the results of confirming changes in the ratio of KI-67⁺, CD62L^{high}, CD44^{high}, and CD4+ T cells in response to external antigen stimulation in animal models (G1 to G5) to which each vaccine formulation was administered, according to an embodiment.
FIG. 10 shows the results of confirming changes in the ratio of KI-67⁺, CD62L^{high}, CD44^{high}, and CD8+ T cell in response to external antigen stimulation in animal models (G1 to G5) administered with each of the vaccine formulation according to an embodiment.
FIG. 11 shows the result of confirming changes in the ratio of KI-67⁺, CD62L^{low}, CD44^{high}, and CD8+ T cell in response to external antigen stimulation in animal models (G1 to G5) to which each vaccine formulation was administered, according to an embodiment.
FIG. 12 shows the results of assessing immunogenicity to Omicron spike protein antigens over time in animal models (G1 to G5) to which each vaccine formulation was administered, according to an embodiment, wherein A of FIG. 12 is the results of confirming the antibody titer levels of Omicron RBD IgG1, and B of FIG. 12 is the results of confirming the antibody titer levels of Omicron RBD IgG2a.
FIG. 13 shows the results of assessing immunogenicity to Wuhan spike protein antigen over time in animal models (G1 to G5) to which each vaccine formulation was administered, according to an embodiment, wherein A of FIG. 13 is the results of confirming the antibody titer levels of Wuhan RBD IgG1, B of FIG. 13 is the results of confirming the antibody titer levels of Wuhan RBD IgG2a, C of FIG. 13 is the results of confirming the antibody titer levels of Wuhan S IgG1, and D of FIG. 13 is the results of confirming the antibody titer levels of Wuhan S IgG2a.
FIG. 14 shows the results of evaluating the neutralizing ability against pseudoviruses in animal models (G1 to G5) to which each vaccine formulation was administered, according to an embodiment, and the result of confirming serum dilution factor (VNT50) for the Vero cell line infected with VSV pseudotyped virus (REVACC SCIENTIFIC).

### Mode for Invention

Hereinafter, a preferred embodiment of the present disclosure will be presented to aid understanding. However, the following embodiments are provided only to aid understanding of the disclosure, and the disclosure is not limited by the following embodiments.

### Example 1. Production of mRNA antigen expression system and comparison of expression levels of target antigen

In this embodiment, it was sought to determine whether the expression level of a target antigen in a cell may be improved using the mRNA antigen expression system according to an embodiment. To this end, an mRNA transcription vector according to an embodiment was produced using luciferase mRNA as the target antigen, and the mRNA molecule produced thereby was transfected into a HEK 293 cell, and the expression level was quantitatively compared.

### 1-1. Production of mRNA transcription vector

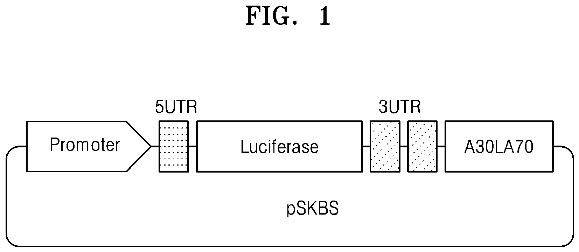

As shown in FIG. 1, an mRNA transcription vector was prepared using as a basic skeleton a structure in which a promoter region sequence, a 5' UTR region sequence, an ORF region sequence, a 3' UTR region sequence, and a poly A-tail region sequence were operably linked sequentially from a 5' end to a 3' end. In this embodiment, a pUC19 plasmid was used to produce a pSKBS plasmid (SEQ ID NO: 8) by inserting a T7 promoter and 5'UTR region sequence (SEQ ID NO: 6) between a Kpnl and BamHI recognition sites (GGTACC ~ GGATCC), and a 3'UTR and A30LA70 (SEQ ID NO: 7) between an Xbal and Hindlll recognition sites (TCTAGA ~ AAGCTT) of the plasmid. Then, to express the target antigen, luciferase mRNA, the luciferase gene (SEQ ID NO: 9) was inserted between the BamHI and Xbal recognition sites (GGATCC ~ TCTAGA) of the above plasmid to produce the pSKBS-Luciferase plasmid (SEQ ID NO: 10). Meanwhile, the genetic information of the main functional structure in the pSKBS-Luciferase plasmid is shown in Table 1 below.

**[Table 1]**

| **Name** | **Sequence (5'-> 3')** | **SEQ ID NO:** |
|---|---|---|
| 5' UTR | ACTCTTCTGGTCCCCACAGACTCAGAGAGAAGCCACC | 1 |
| 3' UTR | AGCTCGCTTGTCAATTTCTATTAAAGGTTCCTTTGTTCCCTAAGTCCA ACTACTAAACTGGGGGATATTATGAAGGGCCTTGAGCATCTGGATT CTGCCTAATAAAAAACATTTATTTTCATTGCATCGATAGCTCGCTTTC | 3 |
| | TTGCTGTCCAATTTCTATTAAAGGTTCCTTTGTTCCCTAAGTCCAACT ACTAAACTGGGGGATATTATGAAGGGCCTTGAGCATCTGGATTCTG CCTAATAAAAAACATTTATTTTCATTGCCCGCGG | |
| Promoter | TAATACGACTCACTATAGGGAGA | 4 |
| A30LA70 | AAAAAAAAAAAAAAAAAAAAAAAAAAAAAAAAGCATATGACTAAAAAAAAA AAAAAAAAAAAAAAAAAAAAAAAAAAAAAAAAAAAAAAAAAAAAAAAAAAA AAAAAAAAAAAAAAAAA | 5 |

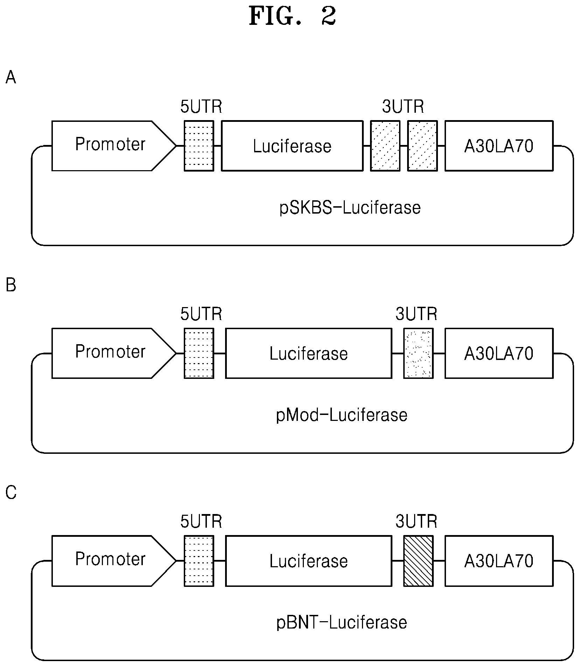

In addition, to compare the expression efficiency of the pSKBS-Luciferase plasmid according to an embodiment with the mRNA antigen, a pMod-Luciferase plasmid including the 5'UTR and 3'UTR of Moderna (Comparative Example 1) was produced, pBNT-Luciferase plasmid (Comparative Example 2) including 5'UTR and 3'UTR of BioNTech, respectively (2004, NAR, A simple, rapid, high-fidelity and cost effective PCR based two process DNA synthesis method for long gene sequence). Specifically, the pMod-Luciferase plasmid was produced using the pUC19 plasmid, after inserting the T7 promoter and the sequence of the 5'UTR region (SEQ ID NO: 11) of Moderna between the Kpnl and BamHI recognition sites (GGTACC ~ GGATCC), the 3'UTR region sequence (SEQ ID NO: 12) of moderna was inserted between the Xbal and Sacll recognition sites (TCTAGA ~ CCGCGG) of the above plasmid, and the luciferase gene was inserted between the BamHI and Xbal recognition sites (GGATCC ~ TCTAGA) of the above plasmid. In addition, the pBNT-Luciferase plasmid also adopted the recognition site used in the production of the pMod-Luciferase plasmid above, and inserting the 5'UTR region sequence (SEQ ID NO: 13) and 3'UTR region sequence (SEQ ID NO: 14) of BioNTech in the same manner. The structure of the mRNA transcription vector produced in this embodiment is shown in FIG. 2.

### 1-2. Evaluation of expression level of target antigen in cells

*E. coli* transformed with the plasmid prepared in Example 1-1 was cultured, the plasmid was isolated from it, and the plasmid was linearized using EcoRV restriction enzyme. The linearized plasmid was recovered by PCI ethanol precipitation and used in an *in vitro* transcription (IVT) reaction for mRNA production. To this end, the linearized plasmid DNA template etc. were mixed according to the conditions described in Table 2 and reacted at 37 °C for 2 hours. After termination of the above reaction, DNase1 at a concentration of 2 KU/mL was added to the reaction and reacted at 37 °C for 15 minutes to remove the linearized plasmid. Then, in order to prepare an mRNA molecule suitable for transfection, the synthesized mRNA was purified using the MEGAclear Transcription Clean-Up Kit (Thermo Fisher). Afterwards, the purified mRNA was transfected into the HEK293 cell line using Lipofectamine^{™} MessengerMAX (Thermo Fisher). 24 hours after transfection, the activity of luciferase expressed in the HEK293 cell line was measured using the ONE-Glo^{™} Luciferase Assay System (Promega), and the relative expression of luciferase was evaluated based on the measured activity. Meanwhile, a group in which transfection was not performed (Mock) was used as a control group.

**[Table 2]**

| **Substance name** | **Applicable concentration** |
|---|---|
| Linearized plasmid DNA template | 0.05 mg/mL |
| ATP | 10 mM |
| CTP | 10 mM |
| UTP | 10 mM |
| GTP | 2 mM |
| ARCA (Anti-Reverse Cap Analog) | 8 mM |
| Dithiothreitol | 10 mM |
| Magnesium chloride | 50 mM |
| RNase inhibitor | 2 KU/mL |
| Pyrophosphatase | 20 unit/mL |
| T7 RNA Polymerase | 300 KU/mL |

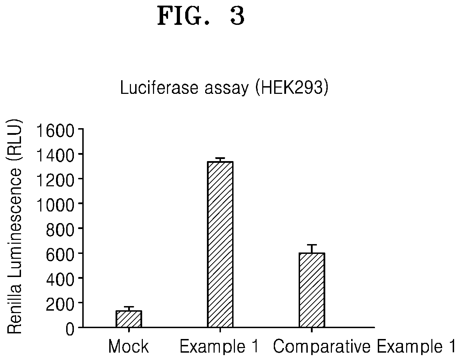

FIG. 3 shows the result of confirming the activity of luciferase after transfection of luciferase mRNA molecule prepared using an mRNA transcription vector according to an embodiment into a HEK 293 cell. As shown in FIG. 3, the Luciferase mRNA molecule prepared using the mRNA transcription vector according to an embodiment exhibited high levels of Luciferase activity upon transfection into host cells, thereby confirming excellent intracellular expression efficiency by the target mRNA antigen.

### Example 2. Efficacy evaluation of immunogenic compositions using mRNA antigen expression system

In this embodiment, it was sought to determine whether the mRNA antigen expression system according to an embodiment may be used to induce an effective immune response in an animal model. To this end, an mRNA transcription vector according to an embodiment was produced using coronavirus spike mRNA as the target antigen, and the produced mRNA molecule was formulated into a mRNA-lipid Nanoparticle (lipid mRNA-LNP) vaccine formulation, which was then administered to an animal model to evaluate its immunogenicity and, more specifically, the level of antibody titer formation.

### 2-1. Production of mRNA transcription vector and target mRNA molecule

The mRNA transcription vector was prepared in a manner similar to Example 1-1 above. Specifically, in this embodiment, the pUC19 plasmid and a T7 promoter (SEQ ID NO: 15) in which the transcription initiation sequence +1 is A and +2 is G were used, followed by insertion of the above promoter and 5'UTR region sequence (SEQ ID NO: 16) between the Kpnl and BamHI recognition sites (GGTACC to GGATCC), 3'UTR and A20 (SEQ ID NO: 17) were inserted between the Xbal and HindIII recognition sites (TCTAGA to AAGCTT) of the above plasmid to produce a pSKBS-AG plasmid (SEQ ID NO: 18). Afterwards, the spike gene (SEQ ID NO: 19) of SARS-CoV2 beta variant virus was inserted between the BamHI and Xbal recognition sites (GGATCC ~ TCTAGA) of the above plasmid to produce pSKBS-Spike(SA)-AG plasmid (SEQ ID NO: 20).

mRNA was produced using the pSKBS-Spike(SA)-AG plasmid produced above. More specifically, *E. coli* transformed with the plasmid prepared above was cultured, the plasmid was isolated from the culture, and the plasmid was linearized using EcoRV restriction enzyme. The linearized plasmids were recovered by PCI ethanol precipitation and used in an *in vitro* transcription reaction for mRNA production. To this end, the linearized plasmid DNA template etc. were mixed according to the conditions described in Table 3 and reacted at 37 °C for 2 hours. Then, after terminating the above reaction, DNase1 at a concentration of 2 KU/mL was added to the reaction and reacted at 37 °C for 15 minutes to remove the linearized plasmid. Then, in order to add a poly A tail to the 3' end of the mRNA, the substances were mixed under the conditions in Table 4 using the Poly (A) Polymerase Tailing Kit (Lucigen) and reacted at 37°C for 1 hour. Afterwards, the synthesized mRNA was purified on AKTA FPLC (GE Healthcare) using a CIMmultus^{®} Oligo dT (Sartorius) column.

**[Table 3]**

| **Substance name** | **Applicable concentration** |
|---|---|
| Linearized plasmid DNA template | 0.05 mg/mL |
| ATP | 10 mM |
| CTP | 10 mM |
| UTP | 10 mM |
| GTP | 10 mM |
| CleanCap-AG (Catalog No: N-7413) | 8 mM |
| Dithiothreitol | 10 mM |
| Magnesium chloride | 50 mM |
| RNase inhibitor | 2 KU/mL |
| Pyrophosphatase | 20 unit/mL |
| T7 RNA Polymerase | 300 KU/mL |

**[Table 4]**

| **Substance name** | **Volume ratio** |
|---|---|
| IVT reaction terminated mRNA | 20 |
| 10X Poly(A) Polymerase Reaction Buffer | 10 |
| 10 mM ATP | 10 |
| Poly (A) Polymerase enzyme (4 KU/mL) | 2 |
| Nuclease free water | 58 |

### 2-2. Production of mRNA-LNP vaccine formulation

The purified mRNA molecule/stock solution of Example 2-1 was formulated into a lipid mixture to prepare mRNA-LNP encapsulated with mRNA in the lipid component. Specifically, the mRNA stock solution was diluted to 0.17 mg/mL with Formulation Buffer, GenVoy-ILM (PRECISION NANOSYSTEMS) was diluted by 1/2 with ethanol, and then each of them was filled into a syringe. Then, the Ignite^{™} equipment was equipped with a microtube cartridge, and the syringe containing the mRNA dilution and the syringe containing the GenVoy-ILM dilution were loaded, and the formulation process was performed under the condition of N/P ratio = 4 by reacting at a volume ratio of 3:1. Afterwards, the reaction solution in which the formulation process was performed was recovered, and buffer exchange was performed with PBS at a volume of more than 20 times using a centrifugal ultrafiltration unit (MWCO 50 kDa), and concentration was performed. Afterwards, the mRNA-LNP stock solution was stored at 4 °C, and after measuring the mRNA content and encapsulation ratio, the mRNA-LNP stock solution was used in animal experiments.

### 2-3. Immunogenicity evaluation

A 0.1 mL injectable formulation including 100 µl of the stock solution including 5 µg or 25 µg of mRNA-LNPs prepared in Example 2-2 above was administered intramuscularly (IM) to an animal model (mouse, BALB/c, female) twice at intervals of 0, 2, or 3 weeks. In addition, serum was isolated from blood samples collected from the animal models before administration and after administration and used as a sample to measure the antibody titer of IgG produced by the vaccine formulation by enzyme-linked immunosorbent assay (ELISA). Specifically, the surface of a 96-well plate was coated with 1 µg/mL of antigen and then incubated at 2 °C to 8 °C for 18 ± 2 hours. The incubated plate was washed and then blocked with BSA solution for 1 hour at room temperature. Afterwards, the plate was washed and a serum dilution solution prepared through serial dilution was dispensed onto the plate. The secondary antibody, Goat Anti-Mouse IgG-Alkaline phosphatase conjugates, was diluted and added to the plate, and then reacted for 2 hours at room temperature. Afterwards, the plate was washed, 1 mg/mL p-nitrophenylamine buffer was added to the plate, and the plate was reacted for another 2 hours at room temperature. Afterwards, the reaction was stopped by adding 3 M NaOH to each well, and the absorbance at 405 nm and 690 nm was measured to calculate the optical density (OD) value of the IgG antibody titer.

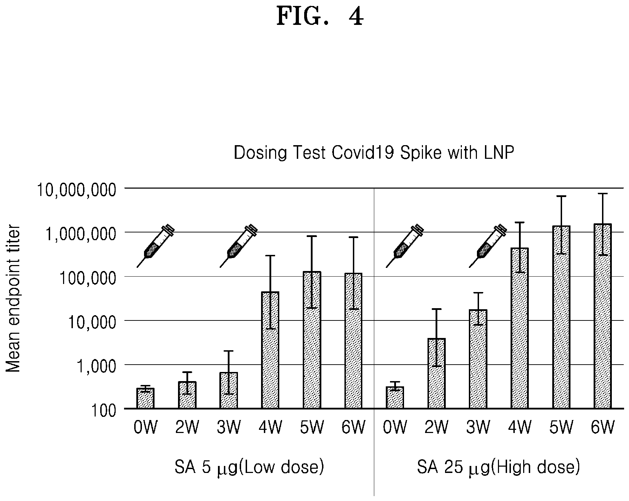

FIG. 4 shows the result of confirming the antibody titer of IgG in the serum of a mouse induced by the formulation after administering a vaccine formulation including spike mRNA molecule in the coronavirus to the mouse according to an embodiment. As shown in FIG. 4, it was found that an effective level of IgG antibody titer was formed by administering the mRNA-LNP vaccine formulation according to an embodiment, and the efficacy was confirmed to be strengthened by repeated administration.

### 2-4. Immunogenicity evaluation by vaccine formulation including various form of modification

In the same manner as Example 2-1 to Example 2-3 above, immunogenicity was evaluated for 1) a vaccine formulation including a full-length Spike mRNA molecule (Spike) or RBD mRNA molecule (RBD) in the coronavirus, 2) a vaccine formulation including a Spike mRNA molecule with a modified poly-A tail sequence, and 3) a vaccine formulation including a mRNA molecule with a modified uridine sequence in the Spike mRNA molecule. Specifically, in this embodiment, the poly-A tail used was a poly-A tail (A30LA70, SAL) in which each a 124 nt long poly-A tail (A124, SA), 30 nt long poly-A, and 70 nt long poly A were connected by a GCAUAUGACU linker, and pseudouridine (Ψ) was applied instead of uridine as a modification of the uridine sequence.

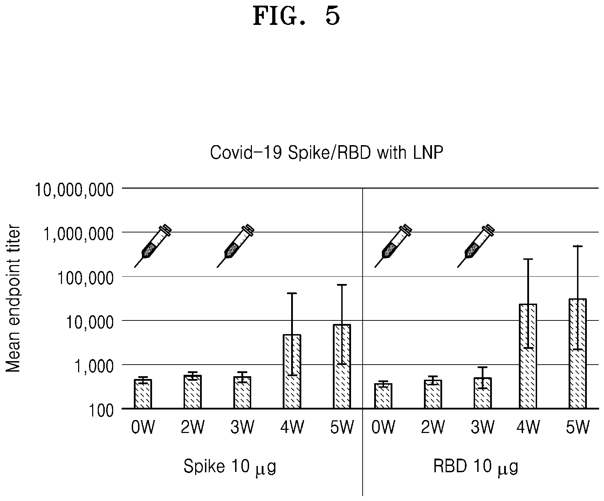

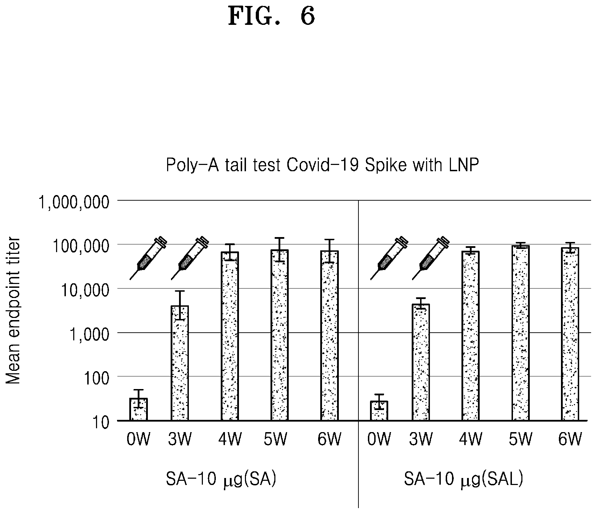

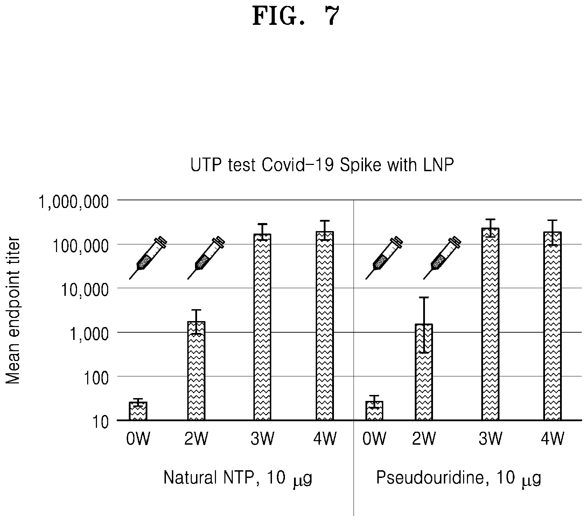

FIG. 5 to FIG. 7 show the results of comparing antibody titer levels of IgG in the serum of a mouse induced by the above compositions after administration to the mouse of a vaccine formulation including a full-length Spike mRNA molecule (Spike) or RBD mRNA molecule (RBD) in a coronavirus, a vaccine formulation including a Spike mRNA molecule with a modified poly-A tail sequence, and a vaccine formulation including a mRNA molecule with a modified uridine sequence in the Spike mRNA molecule, according to an embodiment. As shown in FIG. 5 to FIG. 7, it was confirmed that effective levels of IgG antibody titers were formed in all vaccine formulations including various types of modifications.

### Example 3. Evaluation of efficacy of inducing immune responses using animal model

In this embodiment, it was sought to determine whether an effective immune response may be induced as an effect on a specific external antigen in an animal model using the mRNA antigen expression system according to an embodiment. For this purpose, changes at the cellular level involved in the immune response were evaluated, and cross-reactivity resulting from the characteristics of the coronavirus-derived antigen was confirmed. Meanwhile, the mRNA transcription vector was produced using the pSKBS-AG plasmid (SEQ ID NO: 18) of Example 2-1 above, introducing a SARS-CoV2 Omicron spike-2P prefusion type gene (SEQ ID NO: 21), SARS-CoV2 Omicron spike-6P prefusion type gene (SEQ ID NO: 22), or SARS-CoV2 Wuhan spike-2P prefusion-type gene (SEQ ID NO: 23) between the BamHI and Xbal recognition sites of the plasmid (GGATCC to TCTAGA), wherein as a modification of the uridine sequence, N1-methyl pseudouridine (m1Ψ) was applied instead of uridine in Example 3. The mRNA prepared through this was formulated and 0.1 mL of injectable formulation including 100 µL of a stock solution including 10 µg of mRNA-LNP was administered intramuscularly to an animal model (mouse, BALB/c, female), respectively.

Meanwhile, in this embodiment, an experiment was conducted by classifying the group into a total of 5 groups, as shown in Table 5 below, based on the type of active substance included the administered formulation.

**[Table 5]**

| **Animal test group** | **Active substance** | **Etc.** |
|---|---|---|
| Group 1 | Omicron-S2P | mRNA vaccine |
| Group 2 | Omicron-HexaPro | mRNA vaccine |
| Group 3 | Mock (lipid only) | N/A |
| Group 4 | PBS (buffer only) | N/A |
| Group 5 | Wuhan-S2P | mRNA vaccine |

Thereafter, the animal test group was again classified into a group that did not receive any additional stimulation (Non), a group stimulated with SARS-CoV2 antigen mixture (SARS-CoV-2 (S1), scanning (3629-1), mebtech.com) (Peptide pool), a group stimulated with SARS-CoV2 Wuhan spike antigen (RBD Wuhan), and a group stimulated with SARS-CoV2 Omicron spike antigen (RBD Omicron) according to the type of stimulation by external antigens, and the efficacy of inducing an immune response according to each antigen was evaluated.

### 3-1. Cell-mediated immunoassay (T cell assay)

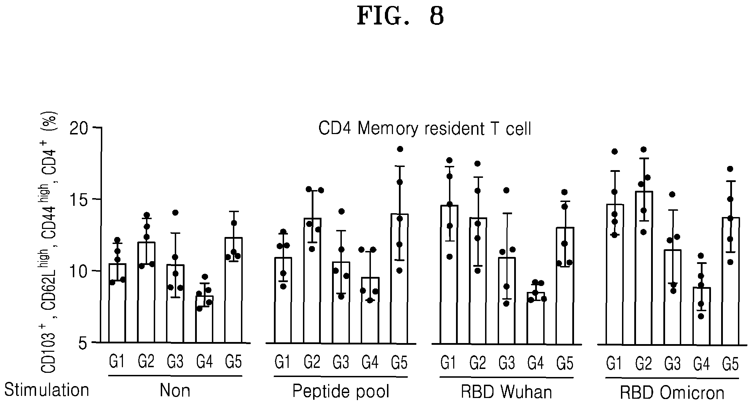

The mRNA-LNP stock solution prepared above was administered intramuscularly (IM) to an animal model (mouse, BALB/c, female). Specifically, two doses were administered at 2-week intervals of week 0 and week 2, and 1 week after the second immunization, the spleen was removed through autopsy, red blood cells were removed from the extracted spleen, and spleen cells were obtained. To analyze the cell lines that react to the antigen among the obtained spleen cells, the spleen cells were stimulated by treatment with Wuhan, Omicron-mutated SARS-CoV2 RBD protein or SARS-CoV2 CD8 epitope prediction peptide pool (Sinobiological), and then labeled with KI-67 dye and CD62L, CD44, and CD8 antibodies. Of the spleen cells prepared as described above, CD4 T cells and CD8 T cells reacting to the antigen were analyzed by fluorescence-activated cell sorting (FACS), and the results are shown in FIG. 8 to FIG. 11.

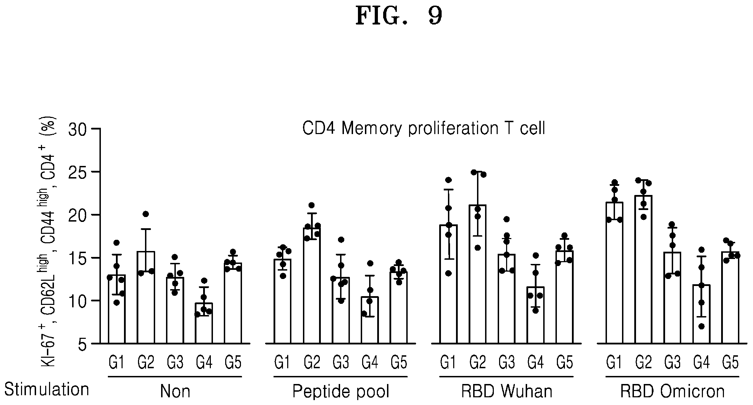

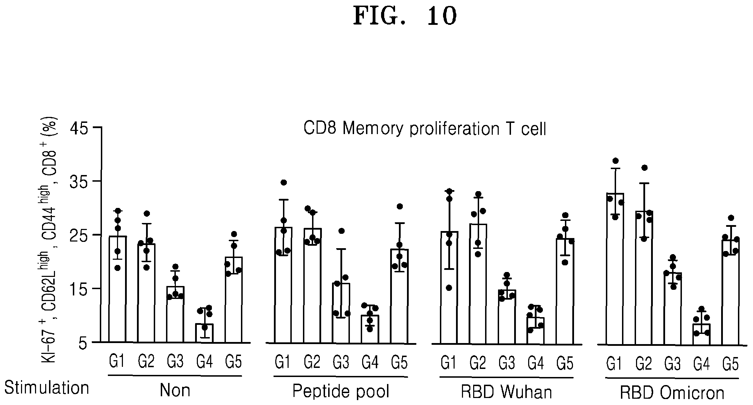

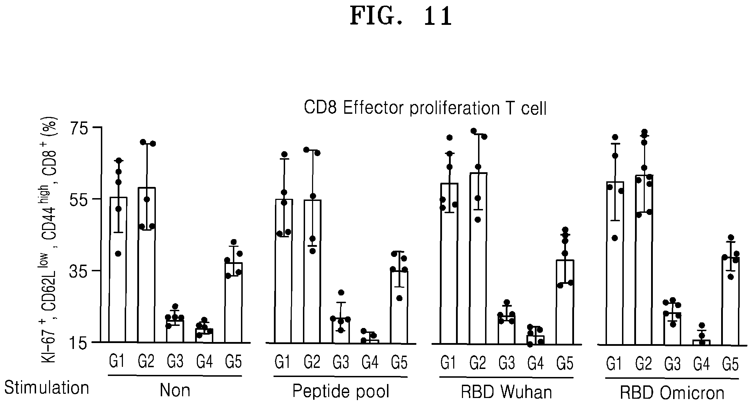

FIG. 8 and FIG. 9 show the results of confirming changes in the ratio of CD103+, CD62L^{high}, CD44^{high}, CD4+ T cells and KI-67⁺, CD62L^{high}, CD44^{high}, CD4+ T cells according to external antigen stimulation in animal models (G1 to G5) administered each vaccine formulation according to an embodiment, FIG. 10 and FIG. 11 show the results of confirming changes in the ratio of KI-67⁺, CD62L^{high}, CD44^{high}, CD8+ T cells and KI-67⁺, CD62L^{low}, CD44^{high}, CD8+ T cells according to external antigen stimulation in animal models (G1 to G5) administered each vaccine formulation according to an embodiment.

As shown in FIG. 8 and FIG. 9 above, it was confirmed that the rates of CD4 memory resident T cells, and CD4 memory proliferation T cells were increased as an immune response to external antigens, in other words, SARS-CoV2 antigen mixture, SARS-CoV2 Wuhan, or SARS-CoV2 Omicron spike antigen. In addition, as shown in FIG. 10 and FIG. 11, it was confirmed that the ratio of CD8 Memory proliferation T cells and CD8 Effector proliferation T cells increased as an immune response to external antigens.

### 3-2. Humoral immunoassay (ELISA assay)

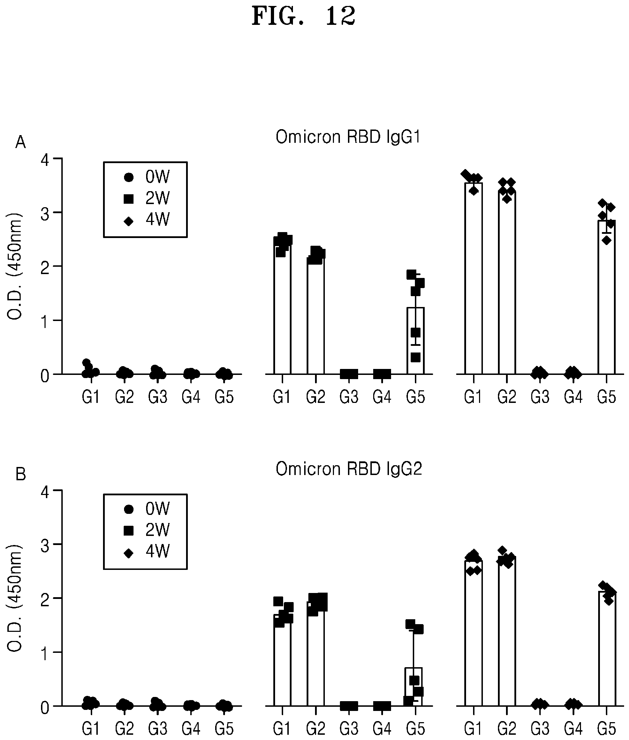

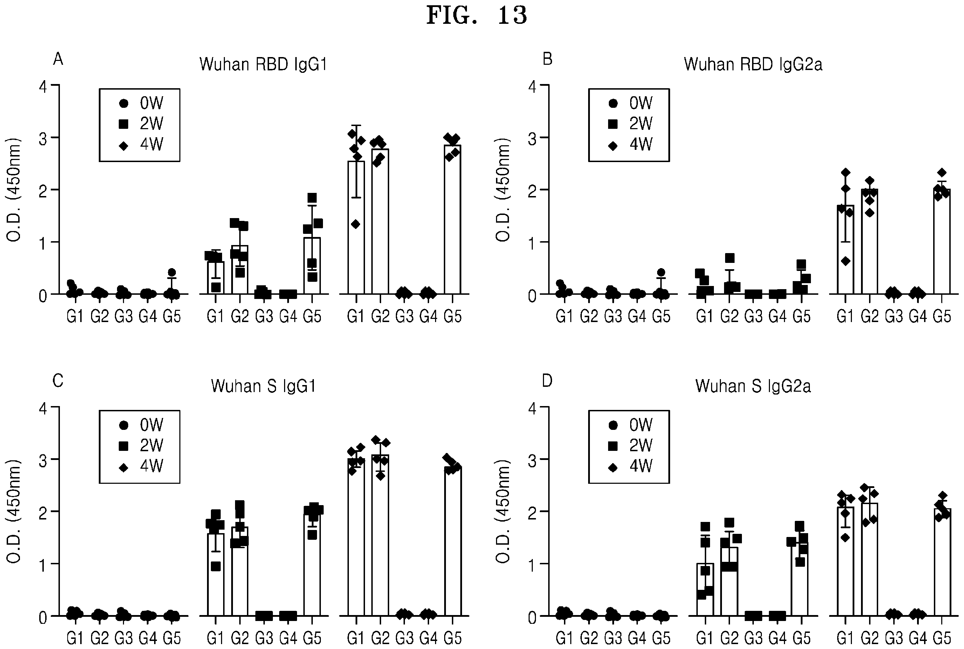

The mRNA-LNP stock solution prepared above was administered intramuscularly (IM) to an animal model (mouse, BALB/c, female). Two doses were administered at 2-week intervals of week 0 and week 2, and serum was separated from blood samples collected before administration and at 2 weeks and 4 weeks after administration. Thereafter, using the separated serum as a sample, the antibody titer of IgG produced by the vaccine Formulation was measured through enzyme-linked immunosorbent assay (ELISA) in the same manner as in Example 2-3 above. Afterwards, the RBD protein and Spike protein of each strain were coated with antigen and the Ig antibody titers were measured, and the results are shown in FIG. 12 and FIG. 13.

FIG. 12 shows the results of evaluating the immunogenicity against Omicron spike protein antigen over time in animal models (G1 to G5) to which each vaccine formulation according to an embodiment was administered, and FIG. 13 shows the results of evaluating immunogenicity against Wuhan spike protein antigen over time in animal models (G1 to G5) to which each vaccine formulation according to an embodiment was administered.

As shown in FIG. 12 and FIG. 13, the vaccine formulation prepared according to an embodiment was confirmed to be cross-reactive to the Wuhan spike protein antigen, based on the antibody titer formation level, across all time points, with Group G1 administered with the SARS-CoV2 Omicron spike mRNA vaccine formulation, and Group G2 administered with the SARS-CoV2 Wuhan spike mRNA vaccine formulation also being cross-reactive to the Omicron spike protein antigen.

### 3-3. Humoral immunoassay (Pseudovirion-based neutralization assay, PBNA)

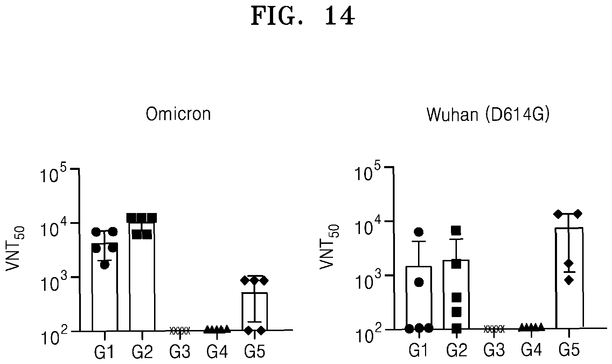

The mRNA-LNP stock solution prepared above was administered intramuscularly (IM) to an animal model (mouse, BALB/c, female). Two doses were administered at 2-week intervals of week 0 and week 2, and serum was separated from blood samples collected at 2 weeks after administration. Afterwards, neutralizing antibody analysis (pseudovirion-based neutralization assay, PBNA) was performed using the separated serum as a sample. SARS-CoV2 Wuhan (D614G), a VSV pseudotyped virus (REVACC SCIENTIFIC) including the Omicron mutant Spike protein and the luciferase gene, was appropriately diluted. Afterwards, this was mixed with the serially diluted serum sample, treated with Vero cell line, and infected for 24 hours, the luciferase activity was measured, and the results are shown in FIG. 14.

FIG. 14 is a graph showing the serum dilution factor (VNT50, 50% neutralization titres) at 50% infection against cultured cells, confirming that the antibodies produced by the groups (G1, G2, and G5) vaccinated with the vaccine formulation have neutralizing ability against pseudoviruses expressing different variants of the spike protein.
The foregoing description of the disclosure is for illustrative purposes only, and one that has ordinary skill in the art to which the disclosure belongs will understand that it may be readily adapted to other specific forms without altering the technical ideas or essential features of the disclosure. Therefore, the embodiments described above should be understood in all respects as illustrative and not restrictive.

## Claims

1. An mRNA transcription vector comprising a promoter region recognized by an RNA polymerase and a gene construct operably linked to the promoter region,
wherein the gene construct comprises: a 5'-untranslated region (5'-UTR) area consisting of SEQ ID NO: 1 or a nucleotide sequence that has 90% or more sequence identity thereto;
an open reading frame (ORF) region comprising a nucleotide sequence encoding a target antigen, operably linked to the 5'-UTR region; and
a 3'-untranslated region (3'-UTR) area comprising two repeats of a monomeric sequence consisting of SEQ ID NO: 2 or a nucleotide sequence that has 90% or more sequence identity thereto, operably linked to the open reading frame region.

2. The mRNA transcription vector of claim 1, wherein the promoter region is recognized by an RNA polymerase of any one of T7 RNA polymerase, T3 RNA polymerase, SP6 RNA polymerase, or mitochondrial polymerase (POLRMT).

3. The mRNA transcription vector of claim 1, wherein the open reading frame region comprises a nucleotide sequence encoding an antigen derived from a pathogen.

4. The mRNA transcription vector of claim 3, wherein the pathogen is selected from the group consisting of a virus, a bacterium, a prion, a fungus, a protozoon, a viroid, and a parasite.

5. The mRNA transcription vector of claim 1, wherein the 3'-UTR region consists of a nucleotide sequence to which the monomeric sequence is directly linked or linked by a linker sequence.

6. The mRNA transcription vector of claim 1, wherein the gene construct additionally comprises a nucleotide sequence transcribed into a 5' cap, operably linked to the 5'-UTR.

7. The mRNA transcription vector of claim 1, wherein the gene construct additionally comprises a nucleotide sequence transcribed as a poly A tail, operably linked to the 3'-UTR region.

8. The mRNA transcription vector of claim 7, wherein the poly A tail consists of 20 to 200 adenines.

9. The mRNA transcription vector of claim 1, wherein the mRNA transcription vector is a plasmid.

10. The mRNA transcription vector of claim 1, wherein the mRNA transcription vector is linearized.

11. The mRNA transcription vector of claim 1, wherein the mRNA transcription vector comprises a promoter region consisting of a nucleotide sequence of SEQ ID NO: 4 or SEQ ID NO: 15 and a gene construct,
wherein the gene construct comprises a 5'-UTR region consisting of the nucleotide sequence of SEQ ID NO: 1; an ORF region operably linked to the 5'-UTR region; and a 3'-UTR region consisting of a nucleotide sequence of SEQ ID NO: 3.

12. A method of preparing an mRNA molecule, comprising a process of performing *in vitro* transcription using the mRNA transcription vector of claim 1 as a template.

13. An mRNA molecule prepared by the method of claim 12.

14. An immunogenic composition comprising the mRNA molecule of claim 13 and a pharmaceutically acceptable excipient as an active ingredient.

15. The immunogenic composition of claim 14, wherein the mRNA molecule forms a complex with at least one or more lipid component to form a liposome, lipid nanoparticle, and/or lipoplex.

16. The immunogenic composition of claim 14, wherein the immunogenic composition is administered intramuscularly or subcutaneously.
